# EUROPEAN PATENT SPECIFICATION

(11) **EP 2 167 093 B1**
(45) Date of publication and mention of the grant of the patent: **18.04.2012**
(21) Application number: 08766063.5
(22) Date of filing: 03.06.2008
(51) Int. Cl.: A61K 31/519, A61K 9/14, A61K 9/28

(54) **PHARMACEUTICAL COMPOSITIONS FOR THE TREATMENT OF CHRONIC HEART FAILURE COMPRISING PYRAZOLOPYRIMIDINONE DERIVATIVE COMPOUND**
PHARMAZEUTISCHE ZUSAMMENSETZUNGEN ZUR BEHANDLUNG VON CHRONISCHER HERZINSUFFIZIENZ MIT EINER PYRAZOLOPYRIMIDINON-DERIVAT-VERBINDUNG
COMPOSITIONS PHARMACEUTIQUES POUR LE TRAITEMENT DE L'INSUFFISANCE CARDIAQUE CHRONIQUE COMPRENANT UN COMPOSÉ DÉRIVÉ DE PYRAZOLOPYRIMIDINONE

(30) Priority: 09.06.2007 KR 20070056392
(43) Date of publication of application: 31.03.2010
(73) Proprietor: Dong-A Pharmaceutical Co., Ltd., Seoul 130-072 (KR)
(72) Inventor: SHIN, Jee-Hyun, Seoul 143-802 (KR); AHN, Gook-Jun, Yongin-si Gyeonggi-do 443-470 (KR); KANG, Kyung-Koo, Suwon-si Gyeonggi-do 443-470 (KR); AHN, Byoung-Ok, Yongin-si Gyeonggi-do 449-813 (KR); YOO, Moo-Hi, Seoul 135-807 (KR)
(74) Representative: Grünecker, Kinkeldey, Stockmair & Schwanhäusser
(86) International application number: PCT/KR2008/003101
(87) International publication number: WO 2008/153282

(56) References cited:
- WO-A1-00/27848
- KR-B1- 100 353 014
- KR-B1- 100 358 083
- KR-B1- 100 395 718
- TAKIMOTO EIKI ET AL: "Chronic inhibition of cyclic GMP phosphodiesterase 5A prevents and reverses cardiac hypertrophy" NATURE MEDICINE, vol. 11, no. 2, February 2005 (2005-02), pages 214-222, XP002587924 ISSN: 1078-8956
- SANDNER P., ET AL.: "PDE5 Inhibitors beyond Erectile Dysfunction" INT. J. IMPOTENCE RES., vol. 19, December 2007 (2007-12), pages 533-543, XP009134960 ISSN: 0955-9930 DOI: 10.138/sj.ijir.3901577

## Description

### [Technical Field]

The present invention relates to a pharmaceutical composition for treating chronic heart failure (CHF) comprising, as an effective ingredient, 5-[2-propyloxy-5-(1-methyl-2-pyrrolidinyl-ethyl-amidosulfonyl)phenyl]-1-methyl-3-propyl-1,6-dihydro-7H-pyrazolo(4,3-d)pyrimidine-7-one.

### [Background Art]

The heart consists of four chambers, two atria (upper chambers) and two ventricles (lower chambers), and four major valves. As the heart beats, the right atrium receives venous blood from the body. The blood then passes through the tricuspid valve to the right ventricle. At the same time, blood oxygenated in the lungs flows into the left atrium and then passes through the mitral valve into the left ventricle. The contraction of the right ventricle propels the blood collected in the right atrium into the lung, in which the venous blood picks up oxygen. Oxygenated blood is returned to the left atrium and flows down into the left ventricle. The left ventricle contracts to pump the blood to the body. Of the four heart valves, two valves prevent blood from flowing backward between an atrium and a ventricle, and the other two valves prevent the backward flow of blood between a ventricle and an artery. The heart beats to supply blood to all parts of the body. The blood pumped from the heart circulates in the body to deliver oxygen and nutrients to tissues and carries waste products away from tissues. The volume of blood expelled by the heart with each beat varies depending on physical activity. A relatively small volume of blood is required for resting time, while a large volume of blood is needed for exercise. In this way, the heart beats slowly or rapidly according to demands for blood, leading to relaxation and contraction of blood vessels.

Heart failure is a pathophysiologic state in which the heart loses its ability to pump a sufficient amount of blood to meet the needs of the body tissues, and is caused by several factors. When the cardiac muscle is not able to contract or relax forcefully enough, blood circulation becomes difficult, and a larger volume of blood accumulates in each chamber of the heart and the lungs. When this happens, the heart tries to compensate for this by becoming enlarged to pump more blood to tissues or organs. When this state lasts for a long time, the compensation effort reaches its limits, causing more severe problems. In particular, when the pumping capacity of the heart weakens gradually, this state is called chronic heart failure. The body's methods of compensating for this condition include increased heart rate, heart enlargement (dilation), and fluid retention. Since cardiac decompensation is accompanied by congestion, chronic heart failure is also referred as congestive heart failure.

According to the American Heart Association, heart failure affects about five million Americans, and about 550000 new cases are diagnosed each year. Also, about 1% of people aged 50 years or older and about 5% of those aged 75 years or older are suffering from heart failure. About 10% of patients diagnosed with heart failure die from heart failure within one year, and 50% of patients die within five years.

Chronic heart failure may be caused by some diseases, and such diseases are deemed as principal causes of the diminished cardiac function. However, other factors may contribute to the development of heart failure in patients who have lived relatively well for a long time even though they have been suffering from a heart failure-causing disease. The common causes of heart failure include excessive afterload and preload, impaired blood flow into the ventricles, defective heart function caused due to myocardial ischemia, and primary myocardial disease. Factors contributing to heart failure include infective endocarditis, acute myocarditis, uncontrolled hypertension, acute myocardial infarction, pulmonary embolism, various infections, anemia, hyperthyroidism, pregnancy, physical and mental strain, excessive alcohol consumption, and excessive eating.

The symptoms of heart failure vary depending on the severity of the illness, but the most common symptom is breathing difficulty. Difficulty in breathing occurs only during exercise in early stages, but occurs even without physical activity with the progression of heart failure. When someone lies down, breathing becomes more difficult due to the difference in the volume of blood entering the heart. Other symptoms include coughing, wheezing, palpitation, nausea, confusion, enuresis, oliguria, systemic edema, ascites, upper abdominal discomfort, pain and swelling, rapid onset of fatigue, and general weakness.

Heart failure is diagnosed through physical examination, based on personal and family history, or by assessing abnormalities in the left ventricle or the valves. Electrocardiography, echocardiography and cardiac catheterization are performed in order to evaluate cardiac function and detect coronary artery disease and myocardial infarction.

The treatment of chronic heart failure is performed with the removal of contributing factors, the treatment of diseases as fundamental causes, the reduction of dietary salt, the reduction of preload using diuretics, and the reduction of afterload using vasodilators. Digoxin or sympathomimetic amines, which act to increase myocardial contractibility, are also useful in the treatment of chronic heart failure.

Vasodilators, such as angiotensin-converting enzyme (ACE) inhibitors, nitrates, and hydralazine, are primarily used for treating chronic heart failure. Thiazide or loop diuretics can be given in early stages of therapy because they can reduce fluid retention. Digitalis glycosides have also been used to treat heart failure. Beta blockers, such as carvedilol, metoprolol and bisoprolol, have been reported to be effective in chronic heart failure.

ACE inhibitors activate cGMP, which induces vascular smooth muscle relaxation, to enlarge arterial blood vessels. Of ACE inhibitors, enalapril, captopril and lisinopril increase the survival rates of patients having chronic heart failure, and quinapril and fosinopril alleviate symptoms in patients having left ventricular dysfunction. In this way, ACE inhibitors reduce morbidity and mortality of heart failure patients due to left ventricular dysfunction, and are thus used to treat chronic heart failure. Thus, losartan, acting on the rennin-angiotensin system (RAS), has been expected to have therapeutic effects on heart failure, and clinical trials to date have supported its therapeutic efficacy on chronic heart failure. However, the use of losartan is restricted in the case of hypotension patients, and may affect renal function and potassium levels in the body. Chronic coughing is observed in 10% of patients. As well, other side effects include dizziness due to low blood pressure, skin rash, and sudden swelling of the lips and cheeks.

Angiotensin II receptor blockers are used in patients who cannot tolerate the side effects of ACE inhibitors. They improve the symptoms of heart failure through an action mechanism similar to that of ACE inhibitors. Trials are underway to examine whether angiotensin II receptor blockers can be used in combination with ACE inhibitors. Angiotensin II receptor blockers do not cause severe side effects when administered in a single dose per day, but rarely cause renal function deterioration.

Beta blockers have been known to worsen the symptoms of heart failure, but are also considered to have beneficial therapeutic effects. They help relax the heart muscle, weaken myocardial contractibility, and reduce additional cardiac strain. The most commonly used beta blockers are carvedilol and metoprolol, which have beneficial actions to prevent the progression of heart failure, shorten hospitalization, and reduce mortality. Beta blockers should be administered starting from a low dosage, and the dosage should be increased gradually over a long period of several months. During several weeks after administration, symptoms often worsen because the oxygen supply to the body decreases. Other side effects include hypotension, breathing difficulty, and nausea.

In addition to the aforementioned drugs, phosphodiesterase III inhibitors, Class III antiarrhythmics, such as amiodarone, implantable cardioverter defibrillators (ICDs), and calcium channel blockers (CCBs), such as amlodipine and felodipine, are used to treat heart failure. ICDs, which have been still studied, have been approved only for use in a high-risk group, and CCBs have been used only in patients having other indications for CCB therapy.

Phosphodiesterase V (PDE-5) inhibitors have been demonstrated to inhibit the enlargement, remodeling and fibrosis of ventricular muscle in mice in which pressure overload was induced by sildenafil (Nature medicine 2005 11(2): 214-222). The effects of PDE-5 inhibitors have been known to be achieved via a mechanism which involves blocking of the activity of PDE-5, catalyzing the breakdown of cyclic guanosine monophosphatase (cGMP), coupled with an intrinsic signaling system in the heart, so as to inhibit myocardial proliferative responses (Journal of Biological Chemistry 2003 278:47694-47699).

The present inventors developed prior to this application 5-[2-propyloxy-5-(1-methyl-2-pyrrolidinyl-ethyl-amidosulfonyl)phenyl]-1-methyl-3-propyl-1,6-dihydro-7H-pyrazolo(4,3-d)pyrimidine-7-one, designated the compound as "udenafil", and reported that the compound has the effect of inhibiting PDE-5 activity (Korean Pat. Registration No. 0353014).

The inventors of this application conducted further research on the compound, and the research resulted in new findings that the udenafil compound (also referred herein to as "a pyrazolopyrimidinone derivative") prevents ventricular cavity enlargement and ventricular wall thinning in chronic heart failure. The findings further include that the pyrazolopyrimidinone derivative suppresses the ventricular expression of atrial natriuretic peptide (ANP) mRNA, and reduces the amount of collagen deposition, as a marker of ventricular fibrosis, in ventricles, thereby leading to the present invention.

### [Disclosure]

### [Technical Problem]

It is therefore an object of the present invention to provide a pharmaceutical composition comprising a pyrazolopyrimidinone derivative as an effective ingredient for a novel use of the compound in the treatment of chronic heart failure.

It is another object of the present invention to provide a pharmaceutical composition for improving depressed cardiac function by suppressing morphological changes in chronically failing hearts, reducing atrial natriuretic peptide (ANP) levels, and inhibiting ventricular fibrosis.

It is a further object of the present invention to provide a pharmaceutical composition for treating chronic heart failure, comprising, as an effective ingredient, a pyrazolopyrimidinone derivative, which has good pharmacokinetic and safety profiles compared to conventional phosphodiesterase-5 (PDE-5) inhibitors.

### [Technical Solution]

In order to accomplish the above objects, the present invention provides a pharmaceutical composition comprising, as an effective ingredient, 5-[2-propyloxy-5-(1-methyl-2-pyrrolidinyl-ethyl-amidosulfonyl)phenyl]-1-methyl-3-propyl-1,6-dihydro-7H-pyrazolo(4,3-d)pyrimidine-7-one (hereinafter, referred to as "a pyrazolopyrimidinone derivative"), represented by Chemical Formula 1, below, for the novel use of the compound in the treatment of chronic heart failure.

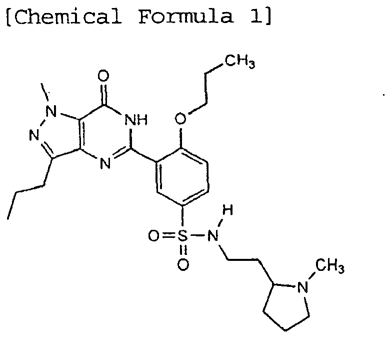

### [Advantageous Effects]

In accordance with the present invention, the pharmaceutical composition comprising the pyrazolopyrimidinone derivative as an effective ingredient suppresses morphological changes in chronically failing hearts. Also, the present composition reduces circulating atrial natriuretic peptide (ANP) levels, which are elevated in a pathophysiologic state of chronic heart failure, and suppresses the cardiac expression of ANP mRNA, thereby being useful as a therapeutic agent for heart failure. Further, the present composition inhibits cardiac fibrosis, thereby improving cardiac systolic and diastolic function.

Also, the pyrazolopyrimidinone derivative has a long in vivo half-life, allowing decreased administration frequency, reaches the maximal plasma level in a short time, has fewer inherent side effects, entails low danger of interaction with other drugs, and has a wide safety margin.

### [Description of Drawings]

FIG. 1 is a graph showing that a pyrazolopyrimidinone derivative according to the present invention reduces serum ANP levels;
FIG. 2 is a graph showing that a pyrazolopyrimidinone derivative according to the present invention reduces ANP mRNA levels in ventricular tissue; and
FIG. 3 is a graph showing that a pyrazolopyrimidinone derivative according to the present invention reduces the amount of collagen deposition in ventricular tissue.

### [Best Mode]

The pyrazolopyrimidinone derivative according to the present invention, which is a phosphodiesterase-5 (PDE-5) inhibitor, exhibits strong and selective inhibitory activity against PDE-5, is rapidly absorbed due to its enhanced solubility, has high bioavailability and large distribution volume, and has an *in vivo* half-life 3 times longer than a drug acting via the same mechanism, sildenafil.

The pyrazolopyrimidinone derivative has the following physicochemical properties: it is difficult to dissolve in water but is readily dissolved in acetic acid, methanol and chloroform; it has a melting point of 158-161 °C; it has a pKa₁ value of about 6.5 and a pKa₂ value of about 12.5; and it is not a hydrate or solvate, but is a white or light white powder.

The pyrazolopyrimidinone derivative is synthesized through a three-step process, as follows.

In brief, The first step is to prepare 4-[2-propyloxy-5-(chlorosulfonyl)benzamido]-1-methyl-3-propyl-5-carbamoyl pyrazole. 4-[2-propyloxy benzamido]-1-methyl-3-propyl-5-carbamoyl pyrazole is mixed with a predetermined amount of chlorosulfonic acid, precooled to 0 °C. The mixture is filtered, and the filtrate is washed and dried to produce 4-[2-propyloxy-5-(chlorosulfonyl)benzamido]-1-methyl-3-propyl-5-carbamoyl pyrazole.

The second step is to prepare 4-[2-propyloxy-5-(1-methyl-2-pyrrolidinylethyl amidosulfonyl)benzamido]-1-methyl-3-propyl-5-carbamoyl pyrazole from the pyrazole compound obtained in the first step. A predetermined amount of a dichloromethane solution of the 4-[2-propyloxy-5-(chlorosulfonyl)benzamido]-1-methyl-3-propyl-5-carbamoyl pyrazole prepared in the first step is mixed with a predetermined amount of 2-(2-aminoethyl)-1-methyl pyrrolidine with stirring at 0 °C. After the reaction is completed, the reaction mixture is diluted in dichloromethane. An organic phase is washed, dried, concentrated and filtered to produce 4-[2-propyloxy-5-(1-methyl-2-pyrrolidinylethyl amidosulfonyl)benzamido]-1-methyl-3-propyl-5-carbamoyl pyrazole.

The third step is to prepare the compound of the present invention, 5-[2-propyloxy-5-(1-methyl-2-pyrrolidinyl-ethyl-amidosulfonyl)phenyl]-1-methyl-3-propyl-1,6-dihydro-7H-pyrazolo(4,3-d)pyrimidine-7-one, from the compound obtained in the second step. A predetermined amount of the pyrazole compound synthesized in the second step is dissolved in t-butanol and mixed with a predetermined amount of potassium t-butoxide under reflux for a predetermined period of time. After the reaction is completed, the reaction mixture is cooled, diluted, washed, and dried. The dried product is subjected to distillation under reduced pressure, solvent removal and silica gel column chromatography to produce a pure pyrazolopyrimidinone derivative according to the present invention.

The present invention relates to a pharmaceutical composition for treating chronic heart failure, and may be described in detail as follows.
1) The present invention provides a therapeutic agent that suppresses cardiac morphological changes caused by heart failure.
2) The present invention provides a therapeutic agent that suppresses the expression of atrial natriuretic peptide (ANP), which is elevated in failing hearts, so as to normalize ANP levels, and that inhibits ventricular fibrosis.
3) The present invention provides a therapeutic agent for chronic heart failure, which has better pharmacokinetic properties and is safer than conventional phosphodiesterase-5 (PDE-5) inhibitors.

The therapeutic agent comprising the pyrazolopyrimidinone derivative according to the present invention as an effective ingredient may be used in general pharmaceutical dosage forms. That is, the pyrazolopyrimidinone derivative of the present invention may be administered in a wide variety of oral and parenteral dosage forms upon clinical application, oral administration being preferred in the present invention. A formulation may be prepared with generally used diluents or excipients, such as fillers, thickeners, binders, humectants, disintegrators and surfactants.

Solid formulations for oral administration may include tablets, pills, powders, granules and capsules, and are prepared by mixing the pyrazolopyrimidinone derivative compound with one or more excipients, such as starch, calcium carbonate, sucrose, lactose and gelatin. Also, the solid formulations may include, in addition to a simple excipient, a lubricant such as magnesium stearate or talc.

Liquid formulations for oral administration may include suspensions, internal solutions, emulsions and syrups. The liquid formulations may include, in addition to commonly used simple diluents, such as water and liquid paraffin, various excipients, which are exemplified by humectants, sweeteners, aromatics and preservatives. Formulations for parenteral administration may include sterile aqueous solutions, non-aqueous solutions, suspensions, emulsions, lyophilized preparations, and suppositories. Non-aqueous solutions and suspensions may be prepared with propylene glycol, polyethylene glycol, vegetable oils such as olive oil, and injectable esters such as ethyl oleate. As a base for suppositories, Witepsol, macrogol, Tween 61, cacao oil, laurin oil and glycerinated gelatin may be used.

The dosage of the pharmaceutical composition of the present invention, comprising the pyrazolopyrimidinone derivative as an effective ingredient, may vary depending on the patient's weight, age, gender and diet, the time and mode of administration, excretion rates, and the severity of illness. Preferably, the present compound may be administered to an adult in a daily dosage of 50 to 200 mg. The daily dosage may be taken in a single dose or may be divided into several doses.

### [Mode for Invention]

A better understanding of the present invention may be obtained through the following examples, which are set forth to illustrate, but are not to be construed as the limit of the present invention.

### EXAMPLE: Evaluation of therapeutic effects of the pyrazolopyrimidinone derivative in an animal model of heart failure

The pyrazolopyrimidinone derivative of Chemical Formula 1 according to the present invention was evaluated to determine whether it has a therapeutic effect on heart failure, as follows.

Male Sprague-Dawley rats, weighing 220-240 g, were randomly divided into three groups: a normal control group (Normal), a heart failure control group (CHF) and a treatment group (CHF plus cpd.1), each group consisting of seven rats. The pyrazolopyrimidinone derivative was orally administered to heart failure-developed animals of the treatment group in a dose of 30 mg/kg.

Normal control animals were subjected to a sham operation. Animals of heart failure control and treatment groups underwent a surgical abdominal incision to create an abdominal aorta-to-inferior vena cava fistula so as to artificially increase blood flow from the inferior vena cava into the right atrium. After 8 weeks, 5 ml/kg of a solvent was orally administered to the normal control and heart failure control groups for 8 weeks, and the pyrazolopyrimidinone derivative was orally administered to the treatment group at a dose of 30 mg/kg for 8 weeks. Then, rats were anesthetized with pentobarbital (50 mg/kg, i.p.), and subjected to M-mode echocardiography in order to evaluate interventricular septum wall thickness (IWT) at end-systole (S) and end-diastole (D) of the left ventricle, posterior left ventricular wall thickness (PWT), left ventricular end-systolic dimension (LVESD), and left ventricular end-diastolic dimension (LVEDD). The relative wall thickness (RWT) was calculated according to the following equation: [IWT(D)+PWT(D)]/LVEDD, and is given in Table 1, below.

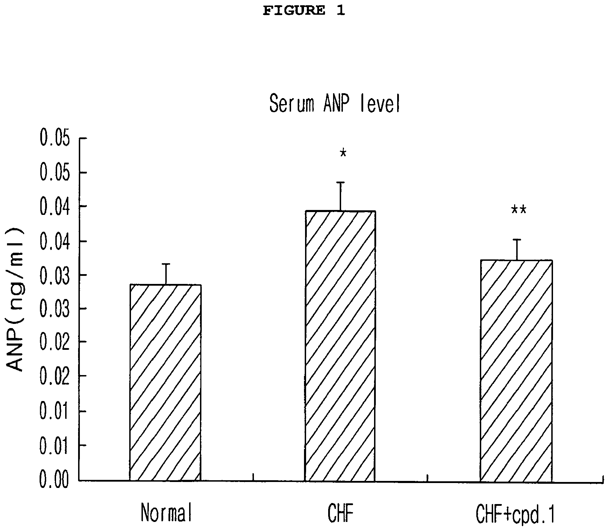

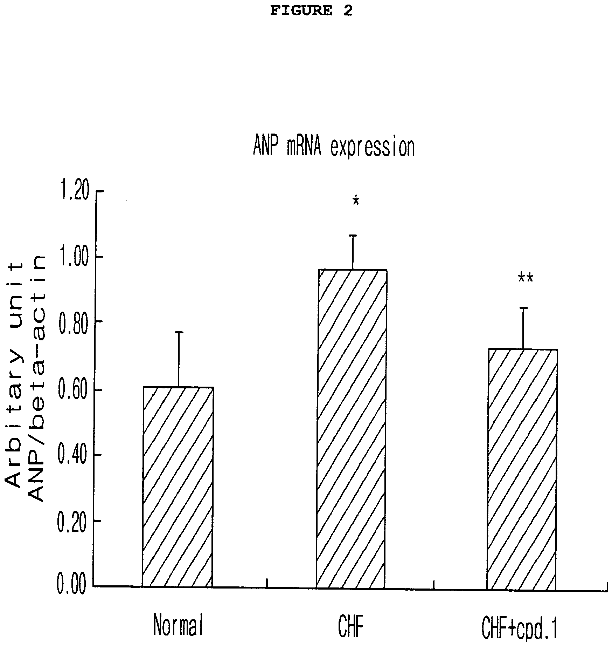

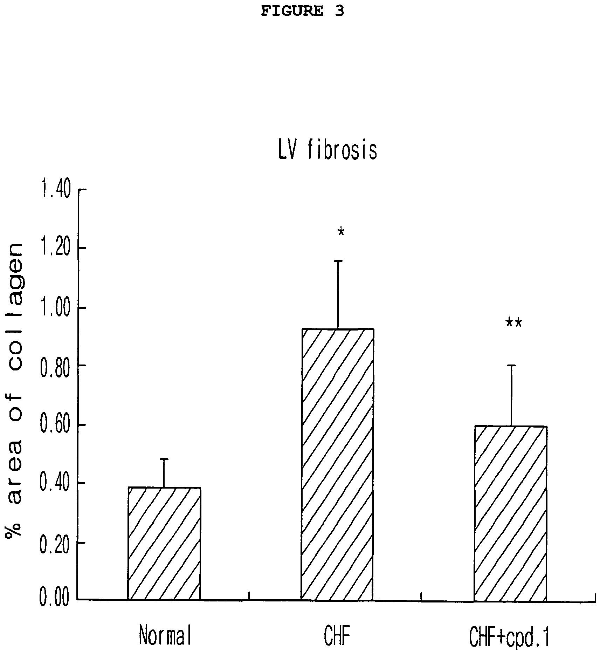

After the echocardiographic measurements, blood samples were collected from abdominal aortas to prepare serum samples. Serum levels of atrial natriuretic peptide (ANP) were determined, and are shown in FIG. 1. 100 mg of each of the left ventricular tissues was stored in liquid nitrogen and then subjected to RT-PCR in order to assess ANP mRNA levels. The measured ANP mRNA levels are shown in FIG. 2. The remaining parts of the tissues were fixed in 10% formalin, and stained with Masson's trichrome to detect collagen fiber in order to assess left ventricular fibrosis, and the results are given in FIG. 3. All values are expressed as mean±SD.

As shown in Table 1, short-axis M-mode echocardiographic images show that the chronic heart failure-induced rats (CHF group) had significantly greater left ventricular end-diastolic and end-systolic dimensions than normal control rats, such as an increase indicating ventricular dilatation, which is a typical sign of heart failure. Interventricular septum wall thickness at end-systole and systolic and diastolic posterior left ventricular wall thickness were significantly smaller, and this decrease results from ventricular cavity enlargement leading to ventricular wall thinning, indicating a clinically significant cardiac change. The relative wall thickness, calculated using the above equation, was also significantly decreased, and these results are consistent with the aforementioned alterations.

In aortocaval fistula rats that received the pyrazolopyrimidinone derivative for an 8-week period (treatment group), left ventricular end-diastolic and end-systolic dimensions were significantly decreased compared to heart failure control rats (CHF group). These results indicate that the ventricular dilatation, found in chronic heart failure, was inhibited through the administration of the pyrazolopyrimidinone derivative. Also, interventricular septum wall thickness at end-systole and end-diastole and posterior left ventricular wall thickness were significantly increased, and the relative wall thickness was significantly increased, compared to the heart failure control group. These results indicate that the pyrazolopyrimidinone derivative inhibits the ventricular dilatation and a decrease in ventricular wall thickness.

FIG. 1 shows the serum levels of atrial natriuretic peptide (ANP). The chronic heart failure control group exhibited significantly increased serum ANP levels (0.040±0.004ng/ml) compared to the normal control group (0.029±0.003ng/ml). This increase was significantly decreased in pyrazolopyrimidinone derivative-received rats (0.033±0.003ng/ml).

FIG. 2 shows ANP mRNA levels in left ventricular tissues. The chronic heart failure control group exhibited significantly increased ANP mRNA levels (0.963±0.114) compared to the normal control group (0.607±0.169). The increased ANP mRNA levels were significantly decreased in pyrazolopyrimidinone derivative-receiving rats (0.739±0.120) compared to the heart failure control group. These results indicate that ANP levels elevated by pathophysiologic changes were decreased to normal levels through the administration of the pyrazolopyrimidinone derivative.

FIG. 3 shows the amount of collagen deposition as a marker of ventricular fibrosis. The chronic heart failure control group exhibited significantly increased collagen deposition (0.928±0.228) compared to the normal control group (0.384±0.103). The increased collagen deposition was significantly decreased through the administration of pyrazolopyrimidinone derivative (0.604±0.214). These results indicate that ventricular fibrosis, causing ventricular systolic and diastolic dysfunction, was decreased by the pyrazolopyrimidinone derivative, thereby enhancing ventricular systolic and diastolic function.

**TABLE 1**

| | | | |
|---|---|---|---|
| M-mode echocardiographic measurements for evaluating chronic heart disease induction in SD rats and therapeutic effects of the pyrazolopyrimidinone derivative on the disease | | | |

| | Normal | CHF | CHF+cpd.1 (30 mg/kg) |
|---|---|---|---|
| IWT(D) (mm) | 1.81±0.20 | 1.56±0.09* | 1.74±0.13** |
| LVEDD (mm) | 8.69±0.56 | 9.75±0.85* | 8.45±0.62** |
| PWT(D) (mm) | 1.83±0.13 | 1.64±0.11* | 1.78±0.04** |
| IWT(S) (mm) | 2.83±0.17 | 2.56±0.11* | 2.74±0.11** |
| LVESD (mm) | 5.43±0.31 | 6.20±0.70* | 4.68±0.38** |
| PWT(S) (mm) | 2.89±0.15 | 2.54±0.18* | 2.80±0.10** |
| RWT | 0.42±0.05 | 0.34±0.02* | 0.42±0.05** |

| | | | |
|---|---|---|---|
| *: The CHF group exhibits a significant difference compared to the normal group (p<0.05). ** : The CHF+cpd.1 group exhibits a significant difference compared to the CHF group (p<0.05). | | | |

### FORMUMATION EXAMPLES: Preparation of pharmaceutical formulations for oral administration

### 1. Preparation of powders

| | |
|---|---|
| The compound of Formula 1 | 2 g |
| Lactose | 1 g |

The above components were mixed and placed in an airtight pack to produce powders.

### 2. Preparation of tablets

| | |
|---|---|
| The compound of Formula 1 | 100 mg |
| Corn starch | 100 mg |
| Lactose | 100 mg |
| Magnesium stearate | 2 mg |

The above components were mixed and tabletted according to a common tablet preparation method to produce tablets.

### 3. Preparation of capsules

| | |
|---|---|
| The compound of Formula 1 | 100 mg |
| Corn starch | 100 mg |
| Lactose | 100 mg |
| Magnesium stearate | 2 mg |

The above components were mixed and loaded into gelatin capsules according to a common capsule preparation method to produce capsules.

## Claims

1. A pharmaceutical composition for use in treating chronic heart failure comprising a pyrazolopyrimidinone derivative represented by the following Chemical Formula 1 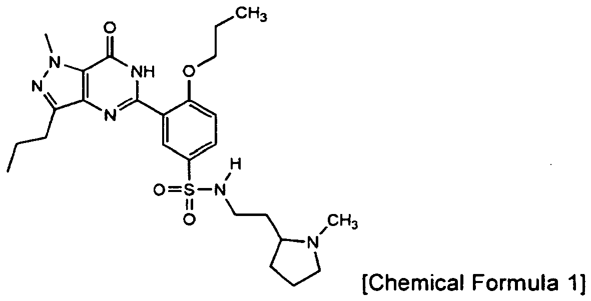 as an effective ingredient.

2. The pharmaceutical composition for use according to claim 1, which inhibits cardiac morphological changes in chronic heart failure.

3. The pharmaceutical composition for use according to claim 1, which inhibits ventricular cavity enlargement and ventricular wall thickness change in chronic heart failure.

4. Use of a pyrazolopyrimidinone derivative represented by the following Chemical Formula 1 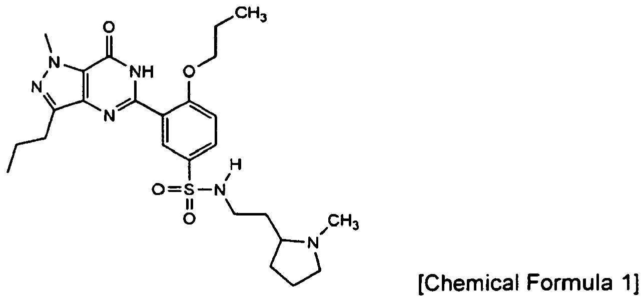 in the manufacture of a pharmaceutical formulation for treating chronic heart failure.

## Patentansprüche

1. Pharmazeutische Zusammensetzung zur Verwendung beim Behandeln von chronischer Herzinsuffizienz, umfassend ein Pyrazolopyrimidinon-Derivat, das von der folgenden chemischen Formel 1 dargestellt wird 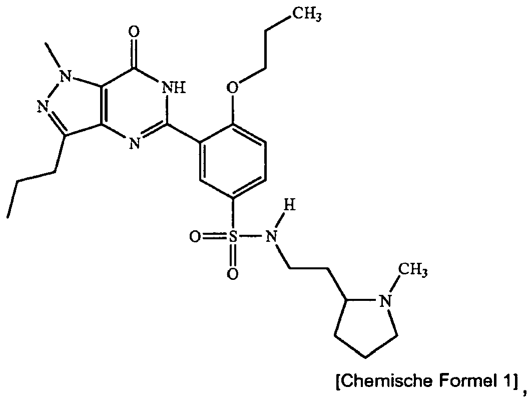 als Wirkstoff.

2. Pharmazeutische Zusammensetzung zur Verwendung gemäß Anspruch 1, welche kardiale morphologische Veränderungen bei chronischer Herzinsuffizienz hemmt.

3. Pharmazeutische Zusammensetzung zur Verwendung gemäß Anspruch 1, welche eine Erweiterung der Ventrikelhöhlen und eine Veränderung der Wanddicke der Ventrikel bei chronischer Herzinsuffizienz hemmt.

4. Verwendung eines Pyrazolopyrimidinon-Derivats, das von der folgenden chemischen Formel 1 dargestellt wird 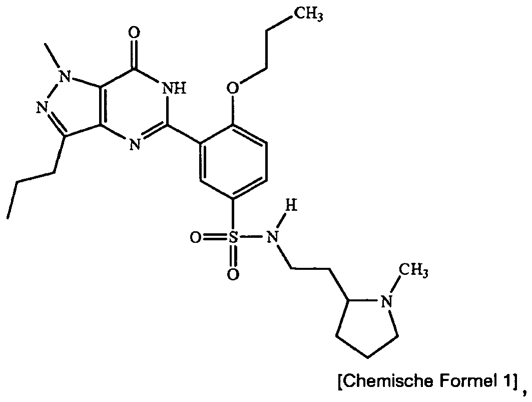 bei der Herstellung einer pharmazeutischen Formulierung zum Behandeln von chronischer Herzinsuffizienz.

## Revendications

1. Composition pharmaceutique pour utilisation dans le traitement de l'insuffisance cardiaque chronique, comprenant, à titre d'ingrédient actif, un dérivé de pyrazolopyrimidinone représenté par la formule chimique 1 suivante :

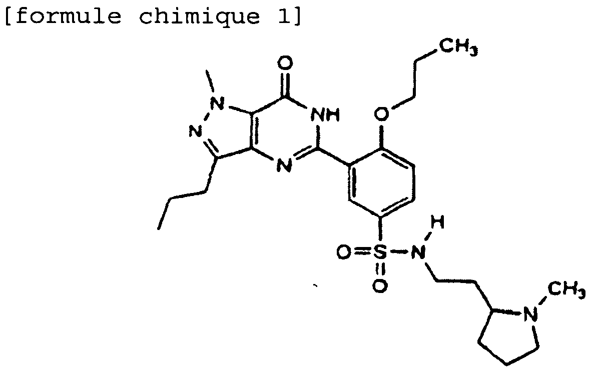

2. Composition pharmaceutique pour utilisation selon la revendication 1, qui inhibe les changements morphologiques cardiaques lors d'une insuffisance cardiaque chronique.

3. Composition pharmaceutique pour utilisation selon la revendication 1, qui inhibe l'augmentation de la taille de la cavité ventriculaire et le changement de l'épaisseur de la paroi ventriculaire lors d'une insuffisance cardiaque chronique.

4. Utilisation d'un dérivé de pyrazolopyrimidinone représenté par la formule chimique 1 suivante : 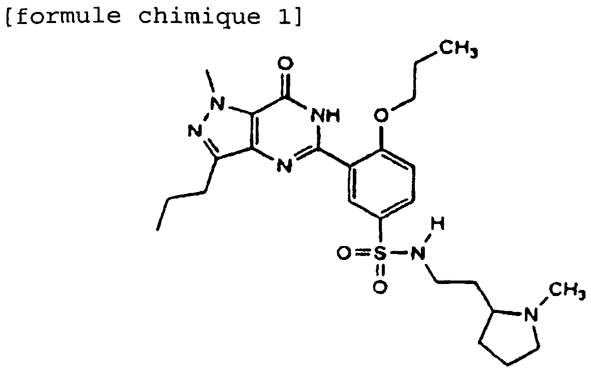 dans la fabrication d'une formulation pharmaceutique destinée au traitement d'une insuffisance cardiaque chronique.
